(19) Europäisches Patentamt
European Patent Office
Office européen des brevets

(11) Veröffentlichungsnummer: **0 114 626**
**B1**

# (12) EUROPÄISCHE PATENTSCHRIFT

(45) Veröffentlichungstag der Patentschrift: **08.11.89**

(51) Int. Cl.⁴: **C 07 D 249/08**, C 05 G 3/08

(21) Anmeldenummer: **84100311.4**

(22) Anmeldetag: **13.01.84**

(54) **Ditriazolylvinyl-phenyl-ketone, Verfahren zu ihrer Herstellung und ihre Verwendung als Nitrifikationshemmer.**

(30) Priorität: **22.01.83 DE 3302098**

(43) Veröffentlichungstag der Anmeldung: **01.08.84 Patentblatt 84/31**

(45) Bekanntmachung des Hinweises auf die Patenterteilung: **08.11.89 Patentblatt 89/45**

(84) Benannte Vertragsstaaten: **AT BE CH DE FR GB IT LI NL SE**

(56) Entgegenhaltungen:
**EP-A- 0 009 298**
**DE-A- 2 745 833**

(73) Patentinhaber: **BASF Aktiengesellschaft, Carl-Bosch-Strasse 38, D-6700 Ludwigshafen (DE)**

(72) Erfinder: **Ruland, Alfred, Dr., Schriesheimer Strasse 11, D-6945 Hirschberg (DE)**
Erfinder: **Reuther, Wolfgang, Dr., Am Pferchelhang 16, D-6900 Heidelberg (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

**EP 0 114 626 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Ditriazolylvinyl-phenyl-ketone, ihre Herstellung und ihre Verwendung als Nitrifikationshemmer.

Ammoniumstickstoff wird im Boden durch Bakterien aus den Gattungen Nitrosomonas und Nitrobacter über Nitritstickstoff zu Nitratstickstoff oxidiert. Das Ausmaß der Nitrifikation ist abhängig von der Bodenart, dem pH-Wert des Bodens, der Bodenfeuchtigkeit und der biologischen Aktivität des Bodens. Da der Nitratstickstoff im Gegensatz zum Ammoniumstickstoff vor allem auch auf leichteren Böden der Auswaschung unterliegt und damit für die Pflanzenernährung nicht mehr verfügbar ist, und die Gefahr einer Nitrat-Anreicherung im Grundwasser besteht, kommt der Nitrifikationshemmung besondere Bedeutung zu.

Ein u.a. für diesen Zweck verwendetes Handelsprodukt ist Dazomet
(3,5-Dimethyltetrahydro-1,3,5-thiadiazin-2-thion).
Literaturhinweis für Dazomet, 1,3-Dichlorpropen, 1,2-Dibrom-3-chlorpropan sowie 2-Chlor-6-trichlor-methylpyridin: Down to Earth 32, 14–17, 1976. Die zuletzt angeführte Verbindung hat auch die Bezeichnung Nitrapyrin und wird als Nitrifikationshemmer unter dem Handelsnamen ®N-Serve vertrieben. Ferner ist als Handelsprodukt noch Dicyandiamid zu nennen, das als Nitrifikationsinhibitor Bedeutung erlangt hat (Landw. Forschung: Sonderheft 27, 74–82, 1972). Schließlich seien Pyrimidin- und Pyrazolderivate erwähnt (vgl. z.B. DE-OS 2 745 833). Diese Substanzen genügen jedoch nicht allen Ansprüchen hinsichtlich ihrer Wirksamkeit und Wirkungsdauer, Wirtschaftlichkeit, Unschädlichkeit oder ihrer anwendungstechnischen Eigenschaften wie Wasserlöslichkeit, Dispergierbarkeit, Dampfdruck usw.

Der Erfindung lag daher die Aufgabe zugrunde, verbesserte Nitrifikationshemmer zu entwickeln, die den bekannten in möglichst vielen der genannten Eigenschaften überlegen sind. Es wurde nun gefunden, daß Ditriazolylvinyl-phenyl-ketone der Formel I

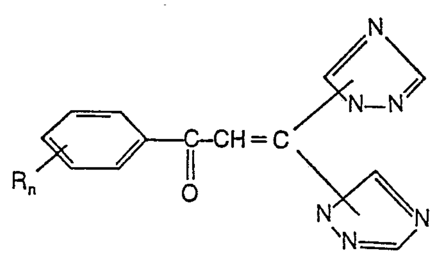

I,

in welcher

R Fluor, Brom, Iod, vorzugsweise Chlor, Alkyl mit 1 bis 6 C-Atomen, vorzugsweise Methyl, Alkoxy mit 1 bis 6 C-Atomen, Trifluormethyl, Cyano, Phenyl oder Phenoxy und

n eine ganze Zahl von 0 bis 5, vorzugsweise 0, 1 oder 2 bedeutet,

und in der die beiden Triazolringe in 4-Stellung oder vorzugsweise in 1-Stellung mit der Vinylgruppe verknüpft sind, überraschend gute Eigenschaften als Nitrifikationshemmer aufweisen.

Es wurde ferner gefunden, daß die Verbindungen der Formel I sehr leicht und in guten Ausbeuten durch Umsetzung von Dihalogenvinylketonen der Formel II, in der R und n die oben angegebenen Bedeutungen haben, und X für Fluor, Iod, vorzugsweise für Chlor oder Brom steht, mit 1,2,4-Triazol erhalten werden.

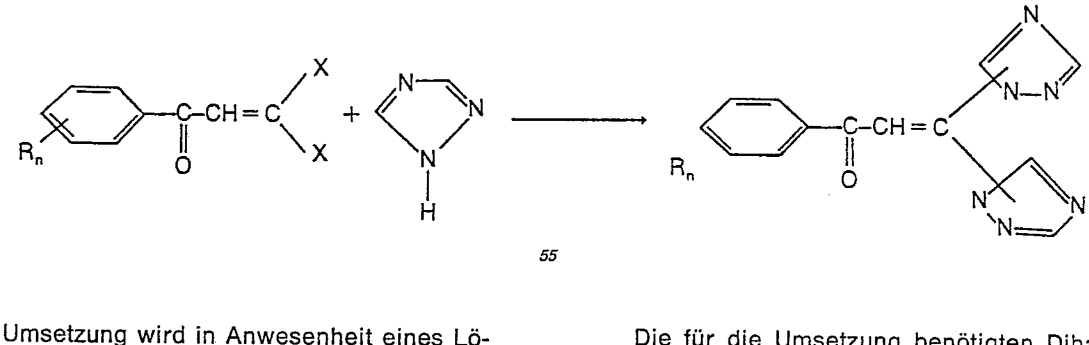

Die Umsetzung wird in Anwesenheit eines Lösungsmittels, z.B. von Dimethylformamid, Dimethylsulfoxid, Hexamethylphosphorsäuretriamid, Tetrahydrofuran, Dioxan oder Acetonitril, und von basischen Substanzen, wie Alkali- oder Erdalkalihydroxiden -carbonaten, -alkoholaten oder -phenolaten, vorzugsweise Natrium- und Kaliumcarbonat, durchgeführt.

Die für die Umsetzung benötigten Dihalogenvinylketone sind bekannt und können nach bekannten Verfahren hergestellt werden (vgl. z.B. Houben-Weyl-Müller, Methoden der organischen Chemie, 4. Auflage, Georg Thieme Verlag, Stuttgart 1968, Bd. 7/4, S. 442).

Die erfindungsgemäßen Substanzen können allein oder im Gemisch mit festen oder flüssigen

Düngemitteln, die Ammoniumstickstoff, Harnstoff oder Ammoniak enthalten, angewendet werden; auch ist die Ausbringung gemeinsam mit Pflanzenbehandlungs- oder Bodenverbesserungsmitteln möglich. Zweckmäßigerweise werden die Wirkstoffe gleichzeitig mit dem Düngemittel ausgebracht. Die Aufwandmengen betragen 0,05 bis 10 kg/ha, vorzugsweise 0,5 bis 3 kg/ha. Bei der mit festen oder flüssigen Düngemitteln kombinierten Anwendung können die Wirkstoffe in Mengen von 0,5 bis 10 Gewichtsprozent, bezogen auf Düngemittelstickstoff, eingesetzt werden.

Die erfindungsgemäßen Nitrifikationshemmer sind sehr wirksam, ungiftig, nicht flüchtig, ausreichend wasserlöslich und stabil. Sie verbleiben lange im Boden, bleiben also lange wirksam. Sie dienen somit nicht nur dem Umweltschutz, indem sie das Auswaschen von Nitrat in das Grundwasser verhindern, sondern auch einer wesentlichen Verbesserung der Ausnutzung des Düngerstickstoffs, insbesondere bei leichteren Böden.

Die in den Beispielen genannten Teile beziehen sich auf das Gewicht. Raumteile (RT) verhalten sich zu Gewichtsteilen wie Liter zu Kilogramm.

Beispiel 1

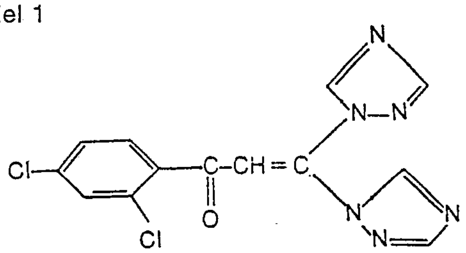

1,1-Bis(1,2,4-triazolyl)-3-(2,4-dichlorphenyl)propen-3-on

27,1 Teile 1,1-Dichlor-3-(2,4-dichlorphenyl)propen-3-on, 13,8 Teile 1,2,4-Triazol und 13,8 Teile Kaliumcarbonat wurden in 300 RT Acetonitril 2 h unter Rückfluß erhitzt. Nach Abkühlen auf Raumtemperatur versetzte man mit 300 RT Wasser, extrahierte zweimal mit je 500 RT Methylenchlorid, trocknete über Natriumsulfat und engte im Vakuum zur Trockne ein.
Ausbeute: 27,8 Teile (80% d.Th.)
Schmp: 200 bis 203 °C

| | | | | |
|---|---|---|---|---|
| $C_{13}H_8Cl_2N_6O$ | Ber. C 46,6 | H 2.7 | N 25.1 | Cl 21.2 |
| (335) | Gef. C 46,8 | H 2.5 | N 24.7 | Cl 20.9 |

Analog Beispiel 1 wurden folgende Produkte erhalten:

2. 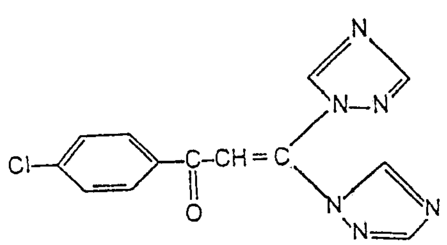

1,1-Bis(1,2,4-triazolyl)-3-(4-chlorphenyl)propen-3-on
Schmp. 202 bis 203 °C

| | | | | |
|---|---|---|---|---|
| $C_{13}H_9ClN_6O$ | Ber. C 51.9 | H 3.0 | N 28.0 | Cl 11.8 |
| (300,5) | Gef. C 52.1 | H 3.0 | N 27.9 | Cl 11.8 |

3. 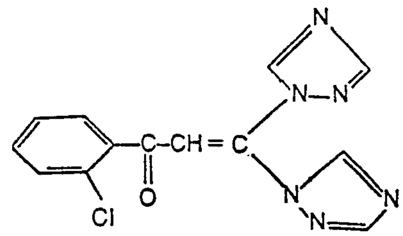

1,1-Bis(1,2,4-triazolyl)-3-(2-chlorphenyl)propen-3-on
Schmp: 168 °C

| | | | | |
|---|---|---|---|---|
| $C_{13}H_9ClN_6O$ | Ber. C 51.9 | H 3.0 | N 28.0 | Cl 11.8 |
| (300,5) | Gef. C 51.9 | H 3.0 | N 27.7 | Cl 11.6 |

4. 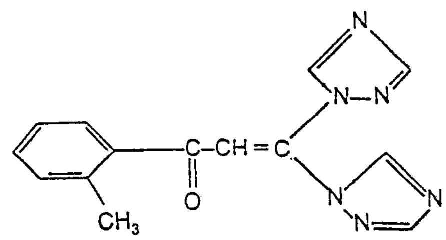

1,1-Bis(1,2,4-triazolyl)-3-(2-methylphenyl)propen-3-on
Schmp: 167 °C

| | | | |
|---|---|---|---|
| $C_{14}H_{12}N_6O$ | Ber. C 60.0 | H 4.3 | N 30.0 |
| (280) | Gef. C 60.0 | H 4.2 | N 29.2 |

Anwendung

Zu 200 g eines lehmigen Sandbodens, dessen Feuchtigkeitsgehalt auf 50% der maximalen Wasserkapazität eingestellt war, wurden 220 mg Ammoniumsulfat gegeben und gründlich vermischt. Danach wurden die Wirkstoffe, in 0,2 ml Aceton gelöst, in Mengen von 1 ppm, bezogen auf feuchten Sandboden, zugesetzt. Nach sorgfältiger Durchmischung wurden die Bodenproben nach Verdunsten des Acetons in mit Aluminiumfolie zur Vermeidung von Wasserverlusten abgedeckten 1 Liter-Gläsern zusammen mit den Kontrollen ohne Wirkstoffzusatz über einen Zeitraum von 28 Tagen bei 21 °C bebrütet. Danach wurden jeweils 2,5 g der Bodenproben in 100 ml-Erlenmeyerkolben eingefüllt und 22,5 ml einer 0,1n Kaliumsulfat-Lösung zugesetzt. Nach 30-minütigem Schütteln wurde abfiltriert, und jeweils 2,5 ml der Bodenauszüge wurden mit 16,25 ml aqua dest. vermischt. Anschließend wurden zum Nachweis der im Bodenauszug noch vorhandenen Ammoniumionen 1,25 ml Neßler-Reagenz hinzugefügt und gründlich geschüttelt. Die Farbveränderungen wurden

dann photometrisch bei einer Wellenlänge von 420 nm gemessen. Anhand von Standardkurven, die durch Messung von Lösungen mit bekannten Ammoniumsulfat-Gehalten ermittelt worden waren, wurden die noch in den Bodenproben vorhandenen Ammoniumsulfat-Mengen bestimmt. Die prozentuale Hemmung der Nitrifikation in den behandelten Bodenproben wurde im Vergleich mit den unbehandelten Bodenproben (ohne Ammoniumsulfat-Zusatz) nach folgender Formel berechnet:

$$\ldots \% \text{ Hemmung der Nitrifikation} = \frac{a-b}{a} \cdot 100$$

a = Nitrifikationsrate von Ammoniumsulfat
b = Nitrifikationsrate von Ammoniumsulfat + Nitrifikationshemmer.

Tabelle

| Wirkstoff Beispiel | | ...% Hemmung der Nitrifikation vier Wochen nach Zugabe von 1 ppm Wirkstoff zum Boden |
|---|---|---|
| 1 | | 79 |
| 2 | | 94 |
| 3 | | 98 |
| 4 | | 98 |
| Vergleichsversuch: (Nr. 95 der DE-OS 27 45 833) | 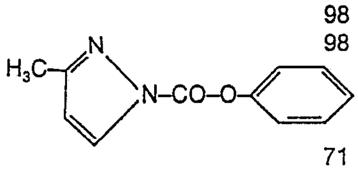 | 71 |

**Patentansprüche**

1. Ditriazolylvinyl-phenyl-ketone der Formel I

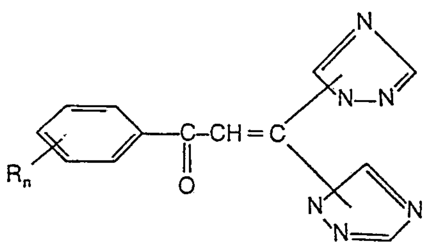
I,

in der
R Halogen, Alkyl mit 1 bis 6 C-Atomen, Alkoxy mit 1 bis 6 C-Atomen, Trifluormethyl, Cyano, Phenyl oder Phenoxy und
n eine ganze Zahl von 0 bis 5 bedeutet,
und in der die beiden Triazolringe wahlweise in 1- oder 4-Stellung mit der Vinylgruppe verknüpft sein können.

2. Ditriazolylvinyl-phenyl-ketone der Formel I, in der
R Chlor oder Methyl und
n 0, 1 oder 2 bedeutet,
und in der die beiden Triazolringe in 1-Stellung mit der Vinylgruppe verknüpft sind.

3. Verfahren zur Herstellung von Verbindungen gemäß Anspruch 1 oder 2, dadurch gekennzeichnet, daß man ein Dihalogenvinyl-phenyl-keton der Formel II

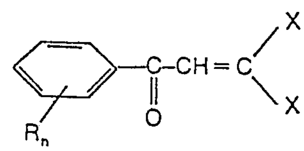
II,

in der R und n die unter Anspruch 1 oder 2 angegebene Bedeutung haben und X für Halogen steht, in Gegenwart eines Lösungsmittels und einer Base bei Temperaturen zwischen 10 und 100 °C mit Triazol umsetzt.

4. Verfahren zur Hemmung der Nitrifikation von Ammoniumstickstoff im Boden, dadurch gekennzeichnet, daß man 0,05 bis 10 kg/ha eines Ditriazolylvinyl-phenyl-ketons der Formel I nach Anspruch 1 oder 2 in den Boden einbringt.

**Claims**

1. A ditriazolylvinyl phenyl ketone of the formula I

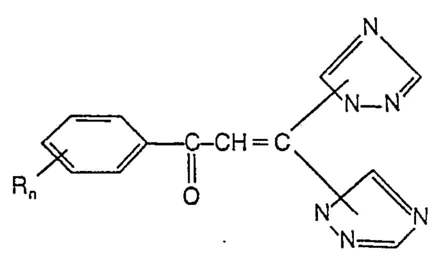
I,

where R is halogen, alkyl of 1 to 6 carbon atoms, alkoxy of 1 to 6 carbon atoms, trifluoromethyl, cyano, phenyl or phenoxy, and n is an integer from 0 to 5, and the two triazole rings can, if desired, be bonded in the 1-position or 4-position to the vinyl group.

2. A ditriazolylvinyl phenyl ketone of the formula I where R is chlorine or methyl and n is 0, 1 or 2, and the two triazole rings are bonded in the 1-position to the vinyl group.

3. A process for the preparation of a compound as claimed in claim 1 or 2, wherein a dihalovinyl phenyl ketone of the formula II

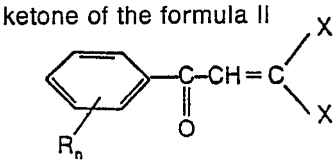 II,

where R and n have the meanings given in claim 1 or 2 and X is halogen, is reacted with triazole in the presence of a solvent and of a base at from 10 to 100 °C.

4. A process for inhibiting nitrification of ammonium nitrogen in the soil, wherein from 0.05 to 10 kg/ha of a ditriazolylvinyl phenyl ketone of the formula I as claimed in claim 1 or 2 are introduced into the soil.

**Revendications**

1. Ditriazolylvinyl-phényl-cétones de formule I

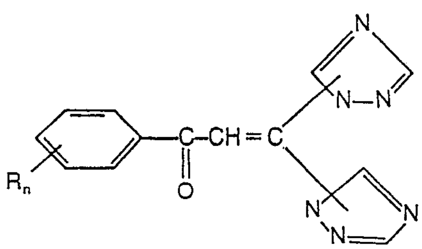 I,

dans laquelle
R représente halogène, alkyle à 1 à 6 atomes C, alcoxy à 1 à 6 atomes C, trifluorométhyle, cyano, phényle ou phénoxy et
n un nombre entier de 0 à 5
et dans laquelle les deux noyaux triazole peuvent être liés au groupe vinyle à volonté en position 1 ou 4.

2. Ditriazolylvinyl-phényl-cétones de formule I, dans laquelle
R représente chlore ou méthyle et
n 0, 1 ou 2
et dans laquelle les deux noyaux triazole sont liés au groupe vinyle en position 1.

3. Procédé de préparation de composés selon la revendication 1 oü 2 caractérisé par le fait que l'on fait réagir avec du triazole une dihalogènovinyl-phényl-cétone de formule II

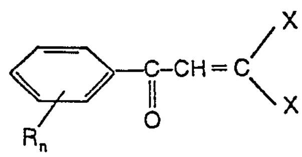 II,

dans laquelle R et n ont les significations données dans les revendications 1 ou 2 et X est mis pour halogène, en présence d'un solvant et d'une base, à des températures comprises entre 10 et 100 °C.

4. Procédé pour inhiber la nitrification de l'azote ammoniacal dans les sols, caractérisé par le fait que l'on introduit dans les sols une ditriazolylvinyl-phényl-cétone de formule I selon la revendication 1 ou 2.